# EUROPÄISCHE PATENTSCHRIFT

(11) **EP 2 986 225 B1**
(45) Veröffentlichungstag und Bekanntmachung des Hinweises auf die Patenterteilung: **16.08.2017**
(21) Anmeldenummer: 14716870.2
(22) Anmeldetag: 14.04.2014
(51) Int. Cl.: A61B 5/18, G06K 9/00, G02B 27/01, G08G 1/16, G02B 27/00

(54) **VERFAHREN ZUR BESTIMMUNG, OB EIN INSASSE EINES FAHRZEUGS RELEVANTE OBJEKTE IM UMFELD DES FAHRZEUGS BEMERKT**
METHOD FOR DETERMINING IF A VEHICLE OCCUPANT IS AWARE OF RELEVANT OBJECTS IN THE SURROUNDINGS OF THE VEHICLE
METHODE PERMETTANT DE DETERMINER SI L'OCCUPANT D'UN VEHICULE A CONSCIENCE DES OBJECTS IMPORTANTS AUX ENVIRONS DU VEHICULE

(30) Priorität: 16.04.2013 DE 102013206739
(43) Veröffentlichungstag der Anmeldung: 24.02.2016
(73) Patentinhaber: Bayerische Motoren Werke Aktiengesellschaft, 80809 München (DE)
(72) Erfinder: KLANNER, Felix, 639664 Singapore (SG); KLÖDEN, Horst, 80797 München (DE)
(86) Internationale Anmeldenummer: PCT/EP2014/057540
(87) Internationale Veröffentlichungsnummer: WO 2014/170278

(56) Entgegenhaltungen:
- EP-A1- 1 484 014
- EP-A1- 1 990 674
- US-A1- 2005 275 718
- US-A1- 2012 212 414

## Beschreibung

Die Erfindung betrifft ein Verfahren zum Bestimmen, ob ein Insasse eines Fahrzeugs eines oder mehrere relevante Objekte im Umfeld des Fahrzeugs bemerkt, eine entsprechende Vorrichtung und ein entsprechendes Computerprogramm.

Heutzutage ist es bekannt, dass die Leistungsfähigkeit von Fahrerassistenz- und Komfortsystemen von Fahrzeugen, insbesondere PKWs, verbessert werden kann, wenn die Blickrichtung oder Kopfpose eines Insassen mit berücksichtigt wird. Zur Erfassung dieser beiden Kenngrößen sind bereits Systeme im Stand der Technik bekannt. In der Regel basieren diese Systeme auf der Auswertung der Aufnahmen von einer oder mehreren Kameras, die den Kopf des Insassen aufnehmen und in den Aufnahmen des Kopfes die Augen, deren Blickrichtung und gegebenenfalls die Kopfpose des Insassen mit bekannten Verarbeitungsmethoden erkennen. Diese Kamera oder Kameras sind typischerweise im Fahrgastraum des Fahrzeugs angeordnet und beispielsweise im Dachhimmel verbaut. Es sind auch Systeme bekannt, in denen die Kamera auf einem Gestell vom Insassen auf dem Kopf getragen wird. Die genannten Systeme werden manchmal auch als Eye-Tracking Systeme bezeichnet.

Ein Nachteil der genannten und heutzutage verfügbaren Systeme zur Erkennung der Blickrichtung und Kopfpose ist, dass die Qualität des Verfolgens der Blickrichtung und der Kopfpose in der Regel eine Kalibrierung des Systems erfordert. Die Qualität der Kalibrierung und deren Aufrechterhaltung hängen dabei stark vom Nutzer ab.

Probleme treten insbesondere bei Brillen-/Kontaktlinsenträgern auf, gerade wenn Brillen auch zum Sonnenschutz dienen und getönte Gläser aufweisen. In diesem Fall kann ein Eye-Tracking unmöglich werden. Weiterhin können nutzerabhängige Probleme während der Fahrt auftreten. Verändert ein Insasse beispielsweise seine Sitzposition oder verstellt den Sitz, verändern sich für die Kalibrierung wichtige Parameter und die Bestimmung der Blickrichtung oder Kopfpose unterliegt dadurch häufig großen Ungenauigkeiten. Eine Neukalibrierung des Systems während der Fahrt ist kaum oder nur erschwert möglich und wird dementsprechend auch nicht ausgeführt. Sensoren in den Sitzen und das Verfolgen der Sitzverstellungen ermöglichen keine hinreichend genaue Anpassung der Kalibrierung. Weiterhin wird die Prozedur des Kalibrierens bei Fahrerwechseln oder nach einer Verstellung des Sitzes als störend empfunden.

Vor kurzem sind Vorrichtungen bekannt geworden, die eine Datenbrille und eine Kamera umfassen. Die Datenbrille umfasst eine halbdurchsichtige Anzeige, mit deren Hilfe dem Benutzer kontaktanaloge Darstellungen angezeigt werden können. Die Kamera ist derart ausgerichtet, dass sie das Blickfeld des Benutzers aufnimmt. Eine Vorrichtung dieser Art ist unter dem Namen "Project Glass" von Google, Inc. bekannt geworden.

Das Dokument EP 1 990 674 A1 ist als nächstliegender Stand der Technik nach dem Oberbegriff des Anspruchs 1 zu sehen und offenbart ein System mit Datenbrille. Das System umfasst einen Sensor um die Umgebungsinformationen zu erfassen und anzuzeigende Informationen zu erzeugen, die dem Benutzer der Datenbrille angezeigt werden.

Das Dokument US 2012/0212414 A1 offenbart eine interaktive Datenbrille, die Inhalt der realen Welt überlagert anzeigt. Dabei wird die Datenbrille aufgrund von Ereignissen oder Sensoreingaben gesteuert.

Aufgabe an den Fachmann ist Bereitstellung eines Verfahrens mit dessen Hilfe bestimmt werden kann, ob ein Insasse eines Fahrzeugs ein Objekt außerhalb des Fahrzeugs wahrgenommen, also bemerkt, hat.

Die Aufgabe wird durch ein Verfahren nach Anspruch 1, eine Vorrichtung nach Anspruch 12 und ein Computerprogramm nach Anspruch 14 gelöst. Vorteilhafte Weiterbildungen sind in den abhängigen Ansprüchen definiert.

In einem Aspekt umfasst ein Verfahren zum Bestimmen, ob ein Insasse eines Fahrzeugs eines oder mehrere relevante Objekte im Umfeld des Fahrzeugs bemerkt hat, Folgendes: Empfangen von Beschreibungen von Objekten im Umfeld des Fahrzeugs, die mithilfe einer Umfeldsensorik des Fahrzeugs erkannt wurden; Bestimmen eines oder mehrerer relevanter Objekte im Umfeld des Fahrzeugs aus den mithilfe der Umfeldsensorik des Fahrzeugs erkannten Objekten; Empfangen einer oder mehrerer Aufnahmen einer Kamera, die an einer Trageeinrichtung, insbesondere einer Brille, montiert ist, die beim bestimmungsgemäßen Tragen durch einen Insassen des Fahrzeugs Aufnahmen macht, die Bereiche des Umfelds des Fahrzeugs umfassen, die im momentanen Blickfeld des Insassen liegen; Erkennen von Objekten in der oder den Aufnahmen der Kamera; Bestimmen, ob das eine oder die mehreren relevanten Objekte im Umfeld des Fahrzeugs in der einen oder den mehreren Aufnahmen der Kamera aufgenommen wurden; Bestimmen, ob das eine oder die mehreren relevanten Objekte im Umfeld des Fahrzeugs von dem Insassen jeweils bemerkt wurden basierend darauf, ob das eine oder die mehreren relevanten Objekte im Umfeld des Fahrzeugs in der einen oder den mehreren Aufnahmen der Kamera aufgenommen wurden, unter der Voraussetzung, dass die Trageeinrichtung bestimmungsgemäß durch den Insassen getragen wird.

Objekte können Fußgänger, Fahrradfahrer, Kinder, PKW, Motorradfahrer, LKW, Fahrstreifenmarkierungen, Leitpfosten oder andere stehende Hindernisse sein. Die Beschreibung von Objekten kann die Position, eine Kategorisierung, die Größe, die Bewegungsrichtung und ähnliches umfassen. Eine Kategorisierung kann sich auf den Typ von Verkehrsteilnehmer beziehen, also beispielsweise Fußgänger, Fahrradfahrer, Kind, PKW, Motorradfahrer oder LKW. Die relevanten Objekte können insbesondere andere Verkehrsteilnehmer sein, mit denen eine Kollision droht, beispielsweise Fußgänger, Radfahrer oder andere motorisierte Verkehrsteilnehmer. Zur Bestimmung der relevanten Objekte kann durch das Fahrzeug eine Situationsanalyse vorgenommen werden. Auf diese Weise kann die Verkehrssicherheit erhöht werden. Allerdings können die relevanten Objekte auch nur Interessenspunkte sein, manchmal points of interest genannt, wie Geschäfte, lnformationstafeln oder Sehenswürdigkeiten. Auf diese Weise kann eine Komfortanwendung realisiert werden.

Ein Insasse kann der Fahrer des Fahrzeugs, aber auch ein Beifahrer oder allgemein ein Mitfahrer sein. Beim Empfangen von Beschreibungen von Objekten im Umfeld des Fahrzeugs kann es sich auch um das Empfangen dieser Beschreibungen innerhalb eines Programms handeln, d.h. das Programm hat die Objekte im Umfeld erkannt und gibt die Beschreibungen nun an ein anderes Modul oder eine andere Funktion weiter.

Das Bestimmen, ob der Insasse das eine oder die mehreren relevanten Objekte bemerkt hat, kann durch eine Wahrscheinlichkeit ausgedrückt werden, um Unterschiede in der menschlichen Wahrnehmung zu berücksichtigen. Für jedes Objekt kann das Bestimmen das Bestimmen der Wahrscheinlichkeit umfassen, dass der Insasse das Objekt bemerkt hat beziehungsweise dass es als bemerkt gilt. Ist diese Wahrscheinlichkeit hinreichend, übersteigt sie also einen Schwellwert, so ist beziehungsweise gilt das entsprechende Objekt als bemerkt. Das Bestimmen, ob der Insasse das eine oder die mehreren relevanten Objekte bemerkt hat, kann mithilfe einer Assoziation der Aufnahme und Erkenntnissen aus der Umfeldwahrnehmung ausgeführt werden.

Die an der Trageeinrichtung montierte Kamera wird hierin nicht als Teil der Umfeldsensorik des Fahrzeugs verstanden. Die Umfeldsensorik des Fahrzeugs umfasst somit nicht die an der Trageeinrichtung montierte Kamera.

Die Schritte des Empfangens einer oder mehrerer Aufnahmen einer Kamera, und des Erkennens von Objekten in der oder den Aufnahmen der Kamera können durch eine Funktion innerhalb des Kamerasystems ausgeführt werden. In diesem Fall wird die Beschreibung der Objekte von dem Kamerasystem zu einer Recheneinheit im Fahrzeug übertragen.

Gemäß dem Verfahren wird also mithilfe einer Kamera das aufgenommen, was der Benutzer sieht, gegebenenfalls nur ein Teil von dem was der Benutzer sieht oder sogar mehr. Aus dieser Aufnahme kann darauf rückgeschlossen werden, ob der Insasse ein Objekt, das durch die im Fahrzeug vorhandene Umfelderkennung erkannt wurde, ebenfalls bemerkt, also wahrgenommen hat. Gegenüber im Stand der Technik bekannten Eye-Tracking Systemen bietet das hier vorgestellte Verfahren somit den Vorteil, dass die Probleme bezüglich der Kalibrierung der Eye-Tracking Systeme nicht bestehen. Auch ist es irrelevant, ob der Insasse eine Brille oder Sonnenbrille trägt.

Vorteilhafterweise ist die Tragevorrichtung selbst eine Brille, die somit verschiedene zusätzliche Funktionen erfüllen kann wie Sehstärkenkorrektur und/oder Sonnenschutz. In einer besonders bevorzugten Ausführungsform umfasst die Brille auch halbdurchlässige Anzeigen, die eine Darstellungsmöglichkeit bieten, wobei gleichzeitig durch die Anzeigen hindurch die Umwelt sichtbar bleibt. Dies ist besonders für kontaktanaloge Darstellungen, auch Augmented Reality genannt, vorteilhaft. Falls die Brille entsprechend ausgestaltet ist, kann sie auch als Designerobjekt wahrgenommen werden.

Vorteilhafterweise ist die Kamera auf der Trageeinrichtung so ausgerichtet, dass der zentrale Bereich des Blickfelds des Fahrers, auch Sichtfeld genannt, aufgenommen wird. Das Blickfeld des Fahrers ist hierin die Umgebung, die der Fahrer optisch wahrnimmt, wenn er geradeaus schaut. Das Blickfeld ändert sich somit nur, wenn der Insasse den Kopf bewegt. Das Blickfeld kann näherungsweise durch einen Winkelbereich um die Richtung herum angegeben werden, in welcher die Augen des Insassen beim Geradeausschauen stehen, hierin auch zentrale Blickrichtung genannt. Der zentrale Bereich kann beispielsweise der Bereich innerhalb der radialen Winkelabweichungen von 20°, 30° oder 40° von der zentralen Blickrichtung sein. Es können natürlich auch nicht nur kreisförmige Bereiche, sondern auch quadratische oder rechteckige Bereiche definiert werden. In einem typischen Fall ist die Kamera auf der Trageeinrichtung derart ausgerichtet, dass die Aufnahmerichtung der Kamera der zentralen Blickrichtung entspricht.

In einer Weiterbildung umfasst das Verfahren ferner: Bestimmen der Position des einen oder der mehreren relevanten Objekte in der oder den Aufnahmen der Kamera; Wobei beim Bestimmen, ob das eine oder die mehreren relevanten Objekte vom Insassen jeweils bemerkt wurden, jeweils die Position des einen oder der mehreren relevanten Objekte in der oder den Aufnahmen der Kamera berücksichtigt. Auf diese Weise kann berücksichtigt werden, in welchem Bereich Objekte von dem Insassen wahrgenommen werden. Es wird also nicht auf die gesamte Aufnahme der Kamera abgestellt und der Aufnahmebereich der Kamera kann beispielsweise größer sein, als der Bereich, in dem Objekte als bemerkt gelten.

In einer Fortführung der vorstehenden Weiterbildung wird beim Bestimmen, ob das eine oder die mehreren relevanten Objekte vom Insassen bemerkt wurden, berücksichtigt, ob die Position des einen oder der mehreren relevanten Objekte jeweils in einem Bereich um die zentrale Blickrichtung des Insassen liegt, wobei dabei ferner insbesondere berücksichtigt wird, ob die Position des einen oder der mehreren relevanten Objekte jeweils mindestens eine vorgegebene Zeitspanne in dem Bereich um die zentrale Blickrichtung des Insassen liegt, was insbesondere mithilfe der Anzahl der Aufnahmen bestimmt wird, in denen die Position des einen oder der mehreren relevanten Objekte jeweils in dem Bereich um die zentrale Blickrichtung des Fahrers liegt. Der Bereich um die zentrale Blickrichtung kann beispielsweise durch eine radiale Winkelabweichung definiert sein, wie 20°, 30° oder 40° von der zentralen Blickrichtung. Es können natürlich auch nicht nur kreisförmige Bereiche, sondern auch quadratische oder rechteckige Bereiche definiert werden. In einer typischen Implementierung entspricht die zentrale Blickrichtung der Aufnahmerichtung der Kamera. Somit wird bestimmt, ob der Insasse überhaupt ausreichend Zeit hatte, ein relevantes Objekt wahrzunehmen, oder ob der Blick nur über die Umgebung geschweift ist, ohne das Objekt zu bemerken. Weiterhin ist es so, dass die Wahrscheinlichkeit der Wahrnehmung (des Bemerkens) durch den Insassen geringer ist, je länger sich das Objekt außerhalb des Bereiches um die zentrale Blickrichtung befand. Die Wahrscheinlichkeit der Wahrnehmung nimmt noch weiter ab, wenn sich das Objekt außerhalb der Aufnahme der Kamera befindet.

In einer Fortführung der vorstehenden Weiterbildung umfasst das Bestimmen, ob das eine oder die mehreren relevanten Objekte vom Insassen jeweils bemerkt wurden, Folgendes: Bereitstellen einer Zuordnung von Wahrscheinlichkeiten der Erkennung eines Objektes durch einen Insassen für mögliche Positionen eines Objektes in Aufnahmen der Kamera; Bestimmen, ob das eine oder die mehreren relevanten Objekte vom Insassen jeweils erkannt wurden anhand der der Position des Objektes zugeordneten Wahrscheinlichkeit oder den den Positionen der Objekte jeweils zugeordneten Wahrscheinlichkeiten; insbesondere wobei die zugeordneten Wahrscheinlichkeiten von der zentralen Blickrichtung zu den Rändern des Blickfeldes hin abnehmen. Es kann dabei ein Schwellwert vorgesehen sein, bei dessen Unterschreitung der zugeordneten Wahrscheinlichkeit ein Objekt als nicht mehr bemerkt gilt.

In einer Fortführung der vorstehenden Weiterbildung werden mehrere insbesondere aufeinanderfolgende Aufnahmen der Kamera empfangen; und das Bestimmen, ob das eine oder die mehreren relevanten Objekte vom Insassen jeweils bemerkt wurden, umfasst Folgendes: Bereitstellen einer Zuordnung von Wahrscheinlichkeitsdichten der Erkennung eines Objektes durch einen Insassen zu möglichen Positionen eines Objektes in Aufnahmen der Kamera; Bestimmen, für das eine oder jedes relevante Objekt, der Wahrscheinlichkeitsdichten, die den Positionen des relevanten Objektes oder der relevanten Objekte in den Aufnahmen jeweils zugeordnet sind; Berücksichtigen der für das eine oder jedes relevante Objekt jeweils zugeordneten Wahrscheinlichkeitsdichten für das Bestimmen, ob das eine oder die mehreren relevanten Objekte vom Insassen jeweils bemerkt wurden, insbesondere die Summe der jeweils zugeordneten Wahrscheinlichkeitsdichten. Auf diese Weise wird berücksichtigt, dass auch ein Objekt, das nur am Rande des Blickfeldes des Insassen positioniert ist, vom Insassen bemerkt wird, wenn es nur lange genug dort positioniert ist. Auch kann eine nur kurze Passage der Position des Objektes durch einen Bereich, dem hohe Wahrscheinlichkeitsdichten zugeordnet sind, und einer Passage der Position des Objektes durch einen Bereich dem geringere Wahrscheinlichkeitsdichten zugeordnet sind, zu einem Bemerken durch den Insassen führen. Mit anderen Worten: Es wird die Trajektorie der Position des Objektes ermittelt und die zugeordneten Wahrscheinlichkeitsdichten über die Trajektorie summiert beziehungsweise integriert. Dabei kann die Verweildauer der Position des Objektes an der jeweiligen Position berücksichtigt werden. Das Ergebnis kann eine Wahrscheinlichkeit der Wahrnehmung des Objektes durch den Insassen sein. Mehrere Aufnahmen der Kamera können zu aufeinanderfolgenden Zeitpunkten (ggf regelmäßig zeitversetzt) aufgenommen worden sein.

Das Verfahren kann ferner umfassen: Ausgeben eines Hinweises oder einer Warnung, wenn bestimmt wird, dass das relevante Objekt oder zumindest eines der relevanten Objekte nicht bemerkt wurde. Der Hinweis kann dem Insassen andeuten, in welcher Richtung bisher nicht bemerkte relevante Objekte sind. Dazu kann die Trageeinrichtung über Anzeigemöglichkeiten verfügen. Auf diese Weise kann das Blickfeld des Insassen und vor allem dessen zentrale Blickrichtung "gesteuert" oder "geregelt". Der Insasse bemerkt aufgrund des Hinweises schließlich das relevante Objekt.

In dem Fall, dass die Trageeinrichtung auch über kontaktanaloge Anzeigemöglichkeiten verfügt, kann das Verfahren umfassen: Ausgeben eines Hinweises oder einer Warnung, wenn bestimmt wird, dass das relevante Objekt oder zumindest eines der relevanten Objekte nicht bemerkt wurde; wobei der Hinweis oder die Warnung insbesondere eines der Folgenden umfasst: Hervorheben des oder der Objekte, die nicht bemerkt wurden jeweils mithilfe eines kontaktanalog dargestellten Hinweises, insbesondere durch ein Symbol oder mit im Vergleich zur restlichen Darstellung der Anzeige unterschiedlichen Transparenz für das oder die Objekte, einem unterschiedlichen Kontrast oder einer bestimmten Farbe.

Zusätzlich oder alternativ kann der Hinweis oder die Warnung umfassen: einen Klang, insbesondere eines dreidimensional erscheinenden Klangs mit an die Warnung oder den Hinweis angepasster Lautstärke und einem Klangmuster ist; und/oder eine Vibration der Trageeinrichtung.

In einer weiteren Ausbildung wird für jedes relevante Objekt, für das bestimmt wurde, dass der Insasse es nicht bemerkt hat, die Richtung ermittelt, in die der Insasse seine zentrale Blickrichtung richten müsste, um das Objekt zu bemerken (es rückt in sein zentrales Blickfeld oder seine zentrale Blickrichtung ist sogar auf das Objekt ausgerichtet). Zur Ermittlung der zentralen Blickrichtung wird die Position der beschriebenen Objekte mit der Position der Objekte in der Aufnahme verglichen. Die Ermittlung der zentralen Blickrichtung kann auch mithilfe anderer Mittel erfolgen, beispielsweise über Gyroskope an der Trageeinrichtung. Diese Richtung kann für jedes Objekt gewichtet werden, und zwar mit dessen Relevanz, insbesondere der Kritikalität oder der Wahrscheinlichkeit einer Kollision. Die gewichteten Richtungen werden als Vektoren summiert um eine gewünschte Richtung zu bestimmen, die durch den Hinweis angedeutet werden soll. Die gewünschte (zentrale Blick-) Richtung, die durch den Hinweis angedeutet werden soll kann auch derart bestimmt werden, dass die größte Anzahl an relevanten Objekten von dem Insassen bemerkt wird. Gerade wenn früh auf ein relevantes Objekt hingewiesen werden soll, muss in typischen Situationen auf mehrere Objekte hingewiesen werden. Da diese in verschiedenen Richtungen vom Insassen aus gesehen liegen können, wird durch dieses Verfahren eine gewünschte Gesamtrichtung ermittelt. In besonders kritischen Situationen kann sich der Hinweis auf die gewünschte Richtung, in die der Insasse seine zentrale Blickrichtung richten soll, nach einem einzigen Objekt richten.

Ein Verfahren gemäß einer Weiterbildung umfasst insbesondere dann wenn bestimmt wird, dass das eine oder die mehreren relevanten Objekte im Umfeld des Fahrzeugs von dem Insassen jeweils nicht bemerkt wurden, Folgendes: Bestimmen der zentralen Blickrichtung des Insassen; Bestimmen einer gewünschten zentralen Blickrichtung des Insassen; Vergleichen der zentralen Blickrichtung mit der gewünschten zentralen Blickrichtung des Insassen; Falls der Vergleich ergibt, dass die zentrale Blickrichtung und die gewünschte zentrale Blickrichtung nicht übereinstimmen: Bestimmen der Änderung der zentralen Blickrichtung, die der Insasse ausführen muss, um seine zentrale Blickrichtung auf die gewünschte zentrale Blickrichtung zu ändern; Ausgeben eines Hinweises auf die Änderung. Auf diese Weise kann die zentrale Blickrichtung des Insassen "gesteuert" werden in dem Sinne, dass dem Insassen Hinweise ausgegeben werden, in welche Richtung der seine zentrale Blickrichtung richten sollte. Der Hinweis kann intuitiv ausgestaltet sein, beispielsweise ein Pfeil in die Richtung, in die der Fahrer seinen Blick richten soll. Generell ist es vorstellbar auch eine Sequenz an gewünschten zentralen Blickrichtungen zu bestimmen. Der Insassen wird dann, nachdem eine gewünschte zentrale Blickrichtung erreicht ist, auf eine weitere zentrale Blickrichtung hingewiesen, solange, bis alle zentralen Blickrichtungen erreicht wurden. Gerade in unübersichtlichen Verkehrssituationen kann dies den Insassen, insbesondere einen Fahrer eines Fahrzeugs, entlasten. Die Sequenz der gewünschten zentralen Blickrichtungen kann aufgrund der Kritikalität, der Wahrscheinlichkeit einer Kollision, oder aufgrund von Straßenverkehrsregeln bestimmt werden. Gewünschte zentrale Blickrichtungen können aufgrund eines einzelnen relevanten Objektes bestimmt werden und in einem einfachen Fall die Richtung sein, in der sich ein relevantes Objekt vom Insassen aus gesehen befindet.

In einer ähnlichen Weiterbildung umfasst das Verfahren ferner: insbesondere falls bestimmt wird, dass das eine oder die mehreren relevanten Objekte im Umfeld des Fahrzeugs von dem Insassen jeweils nicht bemerkt wurden: Bestimmen des Verlaufes der zentralen Blickrichtung des Insassen; Bestimmen des Verlaufes einer gewünschten zentralen Blickrichtung des Insassen; Vergleichen des Verlaufes der zentralen Blickrichtung mit dem Verlauf der gewünschten zentralen Blickrichtung des Insassen; Bestimmen eines Hinweises auf eine Änderung der zentralen Blickrichtung, die der Insasse ausführen muss, um seine zentrale Blickrichtung auf die gewünschte zentrale Blickrichtung zu ändern, unter Berücksichtigung des Vergleichs des Verlaufes der zentralen Blickrichtung mit dem Verlauf der gewünschten zentralen Blickrichtung des Insassen; Ausgeben des Hinweises auf die Änderung.

Durch den Vergleich der Verläufe der zentralen Blickrichtung und der gewünschten zentralen Blickrichtung kann berücksichtigt werden, ob der Insasse auf den Hinweis reagiert. Tut er dies nicht, so kann vorgesehen sein, den Hinweis zu verstärken und den Insassen so zu einer Reaktion zu motivieren. Ebenso kann vorgesehen sein, dass, wenn der Vergleich ergibt, dass der Insasse auf einen vorherigen Hinweis reagiert, der Hinweis vermindert wird (bspw. in der Größe, der Farbe, der Gestalt oder Ähnlichem). Von manchen kann dieses Verfahren als Regelung der zentralen Blickrichtung aufgefasst werden. Auf diese Weise wird der Hinweis je nach Notwendigkeit gestaltet und ermöglicht so die Vermeidung bzw. Verminderung unnötiger Warnungen und die Verstärkung von Warnungen dann, wenn sie nötig sind.

In einer Weiterbildung wird erkannt, dass der Insasse in einen Rückspiegel schaut, was insbesondere durch eine bestimmte Richtung der zentralen Blickrichtung und der entsprechenden Aufnahme durch die Kamera erkannt wird. Die Bestimmung der relevanten Objekte berücksichtigt in diesem Fall die mithilfe des Rückspiegels erkennbaren Objekte. Es kann vorgesehen sein, dass das Verfahren gemäß dem ersten Aspekt oder dessen Weiterbildungen für empfangene Beschreibungen von Objekten im Umfeld des Fahrzeughecks gesondert ausgeführt wird.

Es kann auch eine gridbasierte Lösung zur der Erstellung einer Belegungskarte in der Umfeldwahrnehmung des Fahrzeugs und in der Aufnahme vorgesehen sein. Es erfolgt dabei eine Assoziation der Belegungskarten. Dieses Verfahren der Assoziation von Belegungskarten kann dazu verwendet werden die Richtung herauszufinden, in die der Insasse blickt, also die Richtung der zentralen Blickrichtung bezogen auf ein absolutes Orientierungssystem, beispielsweise in Bezug auf die Längsachse des Fahrzeugs.

In einer Variante der Erfindung werden die Aufnahmen der Kamera über ein Smartphone empfangen. Das Smartphone kann dabei bereits das Erkennen von Objekten in der Aufnahme der Kamera durchführen, es führt also die Vorverarbeitung der Daten durch.

In einem weiteren Aspekt umfasst eine Vorrichtung eine elektronische Recheneinheit, wobei die Vorrichtung dazu eingerichtet ist, eines der obenstehenden Verfahren auszuführen. Gegebenenfalls ist die Vorrichtung dazu eingerichtet ist, die Ausführung der Verfahrensschritte nur zu Veranlassen. Eine elektronische Recheneinheit kann ein Mikrocontroller, ein Computer oder dedizierte Schaltkreise sein. Die Vorrichtung kann programmtechnisch zur Ausführung der Verfahren eingerichtet sein und über die notwendigen Schnittstellen verfügen.

In einem anderen Aspekt umfasst ein Computerprogramm Anweisungen zum Ausführen eines der obenstehenden Verfahren. Die Anweisungen können einen Computer zur Ausführung eines der obenstehenden Verfahren veranlassen.

### KURZE BESCHREIBUNG DER ZEICHNUNGEN

Fig. 1 zeigt schematisch das Umfeld eines Fahrzeugs und die räumlichen Beziehungen zwischen Blickfeld und Objekten.
Fig. 2 zeigt ein Flussdiagramm eines Verfahrens gemäß einem Ausführungsbeispiel.
Fig. 3 zeigt ein Flussdiagramm eines Verfahrens gemäß einem weiteren Ausführungsbeispiel.
Fig. 4 zeigt ein Flussdiagramm eines Verfahrens gemäß einem Ausführungsbeispiel.

### DETAILLIERTE BESCHREIBUNG DES AUSFÜHRUNGSBEISPIELS

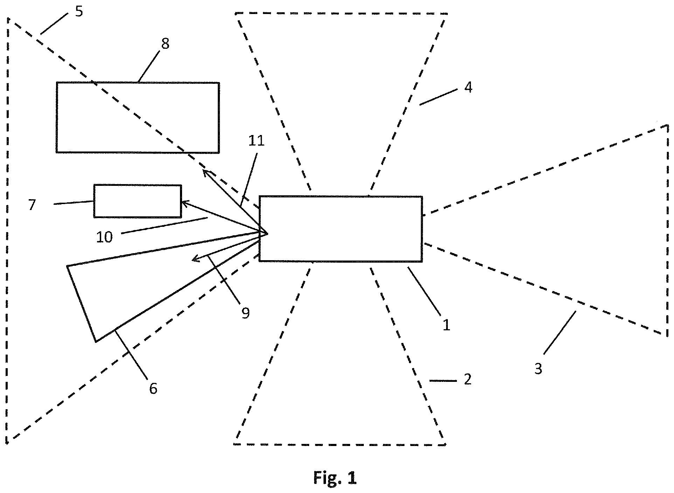

Fig. 1 zeigt schematisch zur Erläuterung das Umfeld eines Fahrzeugs 1 und die räumlichen Beziehungen zwischen dem Blickfeld 6 eines Insassen und Objekten 7 und 8.

Das Fahrzeug 1 befindet sich auf einer Straße und erfasst mit Sensoren das Umfeld. Hierbei wird das seitliche Umfeld links und rechts jeweils in den Bereichen 2 und 4 erfasst. Ferner wird das Umfeld vor dem Fahrzeug in dem Bereich 5 und hinter dem Fahrzeug im Bereich 3 erfasst. Ein Insasse des Fahrzeugs (nicht gezeigt) blickt, bezogen auf die Längsrichtung des Fahrzeugs, leicht nach links. Dabei stellt der durch das Bezugszeichen 6 gekennzeichnete Bereich das zentrale Blickfeld des Fahrers dar. Dieses deckt radiale Winkelabweichungen von ungefähr 11° von der zentralen Blickrichtung 9 des Fahrers ab. Objekte, die in dem zentralen Blickfeld des Fahrers sind, werden von diesem wahrgenommen; er bemerkt diese also. Wie ersichtlich ist, befindet sich ein Fahrradfahrer 7 und eine Sehenswürdigkeit 8 außerhalb des zentralen Blickfeldes 6 des Fahrers. Der Fahrradfahrer 7 und die Sehenswürdigkeit 8 befinden sich in einer Richtung 10 beziehungsweise 11 vom Fahrer. Die Richtungen 9, 10 und 11 können auch als Vektoren aufgefasst werden.

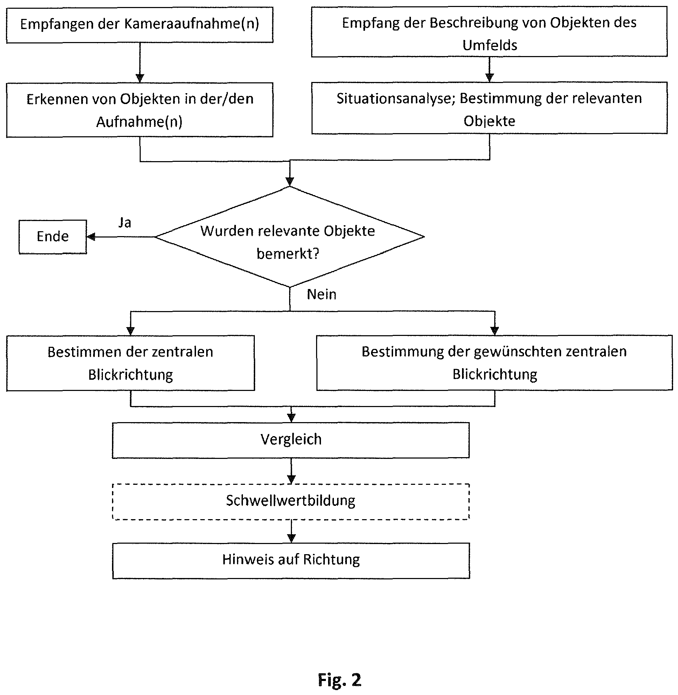

Fig. 2 zeigt ein Flussdiagramm eines Verfahrens gemäß einem Ausführungsbeispiel. Mithilfe der Umfeldsensorik des Fahrzeugs werden Objekte im Umfeld des Fahrzeugs erkannt. Verfahren hierzu sind im Stand der Technik bekannt. Die Objekte werden beschrieben und die Beschreibung wird in einer Funktion empfangen. Dort findet eine Situationsanalyse statt, um relevante Objekte zu bestimmen.

Von der Kamera, die auf einer Datenbrille montiert ist, die der Fahrer trägt, werden Aufnahmen empfangen. Die Kamera ist derart angeordnet, dass die zentrale Richtung der Aufnahme der zentralen Blickrichtung des Insassen entspricht. In den Aufnahmen werden mithilfe von im Stand der Technik bekannter Signalverarbeitung Objekte erkannt und beschrieben.

Im nächsten Schritt wird bestimmt, ob die relevanten Objekte erkannt wurden. Dazu werden die Beschreibungen der Objekte, die in der Kameraaufnahme erkannt wurden, und die empfangenen Beschreibungen der Objekte aufgrund der Umfeldsensorik verglichen. Es wird also bestimmt, ob die relevanten Objekte in der Kameraaufnahme umfasst sind und zwar derart, dass sie im zentralen Blickfeld des Fahrers liegen, also in einem Bereich um den Mittelpunkt der Aufnahme. Wenn dies der Fall ist endet das Verfahren beziehungsweise beginnt von neuem für neue Kameraaufnahmen.

Wurden keines oder nicht alle relevanten Objekte erkannt, so fährt das Verfahren mit der Bearbeitung fort. Es wird die zentrale Blickrichtung des Insassen bestimmt. Dies geschieht über einen Vergleich der Position oder der Positionen eines Objektes beziehungsweise von Objekten in der Kameraaufnahme und gemäß der empfangenen Beschreibung aufgrund der Umfeldsensorik. Weiterhin wird die gewünschte zentrale Blickrichtung des Fahrers ermittelt. Dabei wird für jedes relevante Objekt die Richtung ermittelt, in die der Fahrer blicken müsste, um dieses wahrzunehmen. Aus diesen Richtungen oder der zu den Richtungen korrespondierenden Vektoren wird eine gewünschte zentrale Blickrichtung errechnet. Dies kann die Summation, Mittelwertbildung oder andere Verknüpfung der Vektoren sein, beispielsweise derart, dass der Fahrer die größte Anzahl an relevanten Objekten wahrnimmt. Ist die Wahrnehmung eines Objektes durch den Benutzer besonders wichtig, kann die gewünschte zentrale Blickrichtung durch die Richtung definiert werden, in der sich das betroffene relevante Objekt befindet.

Die bestimmte zentrale Blickrichtung und die gewünschte zentrale Blickrichtung werden verglichen und errechnet, wie der Fahrer seine zentrale Blickrichtung ändern muss um die gewünschte zentrale Blickrichtung zu erreichen. Ein Änderungsvektor wird hierzu bestimmt. In der Datenbrille wird dem Fahrer ein Hinweis angezeigt, wie er seine zentrale Blickrichtung ändern sollte. Dies kann durch explizite Angaben wie Grad geschehen, aber auch durch implizite und den Fahrer intuitiv lenkende Hinweise geschehen. Letztere können beispielsweise für den Fahrer kurzfristig auftauchende Symbole sein. Gemäß typischen menschlichen Reaktionen wird der Fahrer dann seinen Blick in Richtung dieser Symbole lenken und dabei die relevanten Objekte wahrnehmen.

In manchen Implementierungen ist vorgesehen, dass der Betrag des Änderungsvektors einen vorgegebenen Schwellwert überschreiten muss, damit ein Hinweis ausgegeben wird.

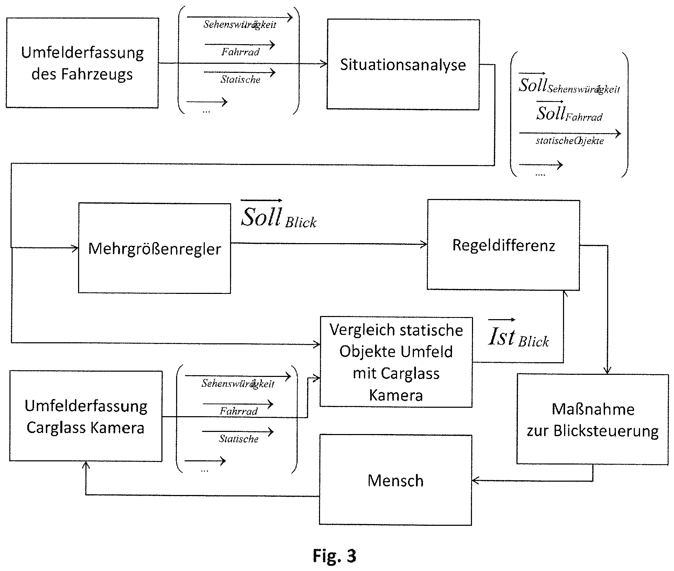

Fig. 3 zeigt ein Flussdiagramm eines Verfahrens gemäß einem weiteren Ausführungsbeispiel. In der Umfelderfassung des Fahrzeugs werden Vektoren zur Richtung von Sehenswürdigkeiten, Fahrrädern, statischen Objekten oder Ähnlichem generiert. Diese Vektoren werden einer Situationsanalyse unterzogen und zentrale Blickrichtungen für die Sehenswürdigkeit, das Fahrrad, statische Objekte oder ähnlichem generiert, in Fig. 3 mit "Soll" bezeichnet. Diese Soll-Vektoren werden einem Mehrgrößenregler zugeführt, der die gewünschte zentrale Blickrichtung bestimmt. Weiterhin werden die Soll-Vektoren dem "Vergleich statischen Objekte Umfeld mit Carglass Kamera" zugeführt, der die aktuelle zentrale Blickrichtung erzeugt. Aufbauend auf der gewünschten zentralen Blickrichtung und der aktuellen zentralen Blickrichtung wird eine Regeldifferenz gebildet. Aus dieser wird eine Maßnahme zur Blicksteuerung abgeleitet, die dem Menschen zugeführt wird. Der Mensch stellt die Basis zur Umfelderfassung durch die Carglass Kamera dar, also der Datenbrille mit integrierter Kamera. Die Carglass Kamera liefert Richtungsvektoren für die Sehenswürdigkeit, das Fahrrad, das statische Objekt, usw.

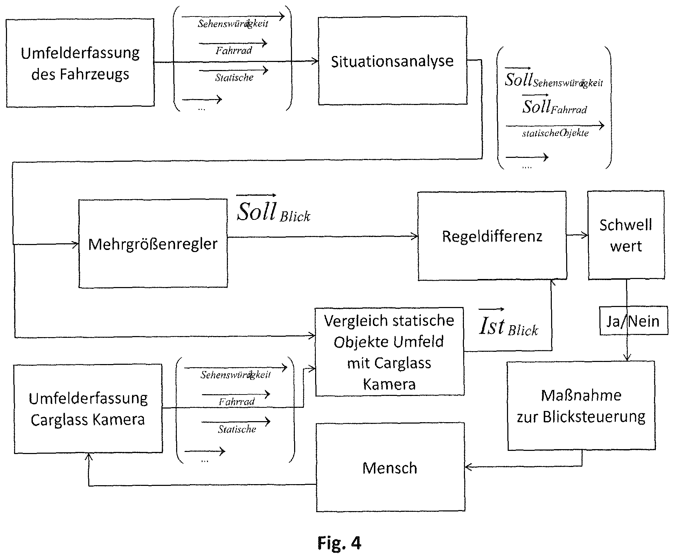

Fig. 4 zeigt ein Flussdiagramm eines Verfahrens gemäß einem Ausführungsbeispiel. Dieses setzt auf dem Flussdiagramm nach Fig. 3 auf. Zusätzlich umfasst das Flussdiagramm in Fig. 4 jedoch einen Schwellwertregler für die Regeldifferenz, der über die Ausführung von Maßnahmen zur Blicksteuerung entscheidet.

## Patentansprüche

1. Verfahren zum Bestimmen, ob ein Insasse eines Fahrzeugs eines oder mehrere relevante Objekte im Umfeld des Fahrzeugs bemerkt hat, umfassend:
Empfangen von Beschreibungen von Objekten im Umfeld des Fahrzeugs, die mithilfe einer Umfeldsensorik des Fahrzeugs erkannt wurden;
Bestimmen eines oder mehrerer relevanter Objekte im Umfeld des Fahrzeugs aus den mithilfe der Umfeldsensorik des Fahrzeugs erkannten Objekten;
Empfangen einer oder mehrerer Aufnahmen einer Kamera, die an einer Trageeinrichtung, insbesondere einer Brille, montiert ist, die beim bestimmungsgemäßen Tragen durch einen Insassen des Fahrzeugs Aufnahmen macht, die Bereiche des Umfelds des Fahrzeugs umfassen, die im momentanen Blickfeld des Insassen liegen;
Erkennen von Objekten in der oder den Aufnahmen der Kamera;
Bestimmen, ob das eine oder die mehreren relevanten Objekte im Umfeld des Fahrzeugs in der einen oder den mehreren Aufnahmen der Kamera aufgenommen wurden;
Bestimmen, ob das eine oder die mehreren relevanten Objekte im Umfeld des Fahrzeugs von dem Insassen jeweils bemerkt wurden, basierend darauf, ob das eine oder die mehreren relevanten Objekte im Umfeld des Fahrzeugs in der einen oder den mehreren Aufnahmen der Kamera aufgenommen wurden, unter der Voraussetzung, dass die Trageeinrichtung bestimmungsgemäß durch den Insassen getragen wird;
Bestimmen der Position des einen oder der mehreren relevanten Objekte in der oder den Aufnahmen der Kamera;
Wobei beim Bestimmen, ob das eine oder die mehreren relevanten Objekte vom Insassen jeweils bemerkt wurden, jeweils die Position des einen oder der mehreren relevanten Objekte in der oder den Aufnahmen der Kamera berücksichtigt wird; **dadurch gekennzeichnet, dass** das Bestimmen, ob das eine oder die mehreren relevanten Objekte vom Insassen jeweils bemerkt wurden, Folgendes umfasst:
Bereitstellen einer Zuordnung von Wahrscheinlichkeiten der Erkennung eines Objektes durch einen Insassen für mögliche Positionen eines Objektes in Aufnahmen der Kamera;
Bestimmen, ob das eine oder die mehreren relevanten Objekte vom Insassen jeweils erkannt wurden anhand der der Position des Objektes zugeordneten Wahrscheinlichkeit oder den den Positionen der Objekte jeweils zugeordneten Wahrscheinlichkeiten.

2. Verfahren nach Anspruch 1, wobei beim Bestimmen, ob das eine oder die mehreren relevanten Objekte vom Insassen bemerkt wurden, berücksichtigt wird, ob die Position des einen oder der mehreren relevanten Objekte jeweils in einem Bereich um die zentrale Blickrichtung des Insassen liegt,
wobei dabei ferner insbesondere berücksichtigt wird, ob die Position des einen oder der mehreren relevanten Objekte jeweils mindestens eine vorgegebene Zeitspanne in dem Bereich um die zentrale Blickrichtung des Insassen liegt,
was insbesondere mithilfe der Anzahl der Aufnahmen bestimmt wird, in denen die Position des einen oder der mehreren relevanten Objekte jeweils in dem Bereich um den die zentrale Blickrichtung des Insassen liegt.

3. Verfahren nach Anspruch 1, wobei die zugeordneten Wahrscheinlichkeiten vom Mittelpunkt des Blickfeldes zu den Rändern des Blickfeldes hin abnehmen.

4. Verfahren nach Anspruch 1,
wobei mehrere insbesondere aufeinanderfolgende Aufnahmen der Kamera empfangen werden;
wobei das Bestimmen, ob das eine oder die mehreren relevanten Objekte vom Insassen jeweils bemerkt wurden, Folgendes umfasst:
Bereitstellen einer Zuordnung von Wahrscheinlichkeitsdichten der Erkennung eines Objektes durch einen Insassen zu möglichen Positionen eines Objektes in Aufnahmen der Kamera;
Bestimmen, für das eine oder jedes relevante Objekt, der Wahrscheinlichkeitsdichten, die den Positionen des relevanten Objektes oder der relevanten Objekte in den Aufnahmen jeweils zugeordnet sind;
Berücksichtigen der für das eine oder jedes relevante Objekt jeweils zugeordneten Wahrscheinlichkeitsdichten für das Bestimmen, ob das eine oder die mehreren relevanten Objekte vom Insassen jeweils bemerkt wurden, insbesondere die Summe der jeweils zugeordneten Wahrscheinlichkeitsdichten.

5. Verfahren nach einem der vorhergehenden Ansprüche, ferner umfassend, insbesondere falls bestimmt wird, dass das eine oder die mehreren relevanten Objekte im Umfeld des Fahrzeugs von dem Insassen jeweils nicht bemerkt wurden:
Bestimmen der zentralen Blickrichtung des Insassen;
Bestimmen einer gewünschten zentralen Blickrichtung des Insassen;
Vergleichen der zentralen Blickrichtung mit der gewünschten zentralen Blickrichtung des Insassen;
Falls der Vergleich ergibt, dass die zentrale Blickrichtung und die gewünschte zentrale Blickrichtung nicht übereinstimmen:
Bestimmen der Änderung der zentralen Blickrichtung, die der Insasse ausführen muss, um seine zentrale Blickrichtung auf die gewünschte zentrale Blickrichtung zu ändern;
Ausgeben eines Hinweises auf die Änderung.

6. Verfahren nach einem der vorhergehenden Ansprüche, ferner umfassend:
insbesondere falls bestimmt wird, dass das eine oder die mehreren relevanten Objekte im Umfeld des Fahrzeugs von dem Insassen jeweils nicht bemerkt wurden:
Bestimmen des Verlaufes der zentralen Blickrichtung des Insassen;
Bestimmen des Verlaufes einer gewünschten zentralen Blickrichtung des Insassen;
Vergleichen des Verlaufes der zentralen Blickrichtung mit dem Verlauf der gewünschten zentralen Blickrichtung des Insassen;
Bestimmen eines Hinweises auf eine Änderung der zentralen Blickrichtung, die der Insasse ausführen muss, um seine zentrale Blickrichtung auf die gewünschte zentrale Blickrichtung zu ändern, unter Berücksichtigung des Vergleichs des Verlaufes der zentralen Blickrichtung mit dem Verlauf der gewünschten zentralen Blickrichtung des Insassen;
Ausgeben des Hinweises auf die Änderung.

7. Verfahren nach einem der vorhergehenden Ansprüche, ferner umfassend:
Ausgeben eines Hinweises oder einer Warnung, wenn bestimmt wird, dass das relevante Objekt oder zumindest eines der relevanten Objekte nicht bemerkt wurde.

8. Verfahren nach einem der vorhergehenden Ansprüche, wobei die Trageeinrichtung eine Brille ist.

9. Verfahren nach Anspruch 8, wobei die Brille eine Anzeige umfasst, die dazu geeignet ist dem Träger der Brille kontaktanaloge Darstellungen anzuzeigen.

10. Verfahren nach Anspruch 9, ferner umfassend:
Ausgeben eines Hinweises oder einer Warnung, wenn bestimmt wird, dass das relevante Objekt oder zumindest eines der relevanten Objekte nicht bemerkt wurde;
wobei der Hinweis oder die Warnung insbesondere eines der Folgenden umfasst:
Hervorheben des oder der Objekte, die nicht bemerkt wurden jeweils mithilfe eines kontaktanalog dargestellten Hinweises, insbesondere durch ein Symbol oder mit im Vergleich zur restlichen Darstellung der Anzeige unterschiedlichen Transparenz für das oder die Objekte, einem unterschiedlichen Kontrast oder einer bestimmten Farbe.

11. Verfahren nach Anspruch 7, wobei der Hinweis oder die Warnung umfasst:
einen Klang, insbesondere eines dreidimensional erscheinenden Klangs mit an die Warnung oder den Hinweis angepasster Lautstärke und einem Klangmuster ist; und/oder
eine Vibration der Trageeinrichtung.

12. Vorrichtung, umfassend eine elektronische Recheneinheit, wobei die Vorrichtung dazu eingerichtet ist, ein Verfahren nach einem der Ansprüche 1 bis 7 auszuführen.

13. Vorrichtung nach Anspruch 12, die weiterhin dazu eingerichtet ist, die Ausführung der Verfahrensschritte nach einem der Ansprüche 8 bis 11 zu veranlassen.

14. Computerprogramm, umfassend Anweisungen zum Ausführen eines Verfahrens nach einem der Ansprüche 1 bis 11.

## Claims

1. A method for determining whether an occupant of a vehicle has noticed one or more relevant objects in the surroundings of the vehicle, comprising:
receiving descriptions of objects in the surroundings of the vehicle, which were recognised with the aid of a surroundings sensor system of the vehicle;
determining one or more relevant objects in the surroundings of the vehicle from the objects recognised with the aid of the surroundings sensor system of the vehicle;
receiving one or more images from a camera, which is mounted on a support device, more especially spectacles or goggles, which, when worn as intended by an occupant of the vehicle, captures images, which comprise regions of the surroundings of the vehicle lying in the current field of view of the occupant;
recognising objects in the image or images of the camera;
determining whether the one or more relevant objects in the surroundings of the vehicle were captured in the one or more images of the camera;
determining whether the one or more relevant objects in the surroundings of the vehicle were noticed in each case by the occupant, based on whether the one or more relevant objects in the surroundings of the vehicle were captured in the one or more images of the camera, on the condition that the support device is worn as intended by the occupant;
determining the position of the one or more relevant objects in the image(s) of the camera;
wherein, when determining whether the one or more relevant objects were noticed in each case by the occupant, the position of the one or more relevant objects in the image(s) of the camera is taken into account;
**characterised in that** determining whether the one or more relevant objects was noticed in each case by the occupant comprises the following:
providing an allocation of probabilities of the recognition of an object by an occupant for possible positions of an object in images of the camera;
determining whether the one or more relevant objects were recognised in each case by the occupant with the aid of the probability allocated to the position of the object or the probabilities allocated in each case to the positions of the objects.

2. A method according to claim 1, wherein when determining whether the one or more relevant objects were noticed by the occupant, it is taken into account whether the position of the one or more relevant objects in each case lies in a region around the central viewing direction of the occupant, wherein, furthermore, it is taken into account here, more especially, whether the position of the one or more relevant objects in each case lies for at least a predetermined timespan in the region around the central viewing direction of the occupant, which is determined, more especially, with the aid of the number of images, in which the position of the one or more relevant objects in each case lies in the region around the central viewing direction of the occupant.

3. A method according to claim 1, wherein the allocated probabilities decrease from the centre point of the field of view to the edges of the field of view.

4. A method according to claim 1, wherein a plurality of, more especially, consecutive images of the camera are received;
wherein determining whether the one or more relevant objects was noticed in each case by the occupant comprises the following:
providing an allocation of probabilities of the recognition of an object by an occupant to possible positions of an object in images of the camera;
determining, for the one or each relevant object, the probability densities, which are in each case allocated to the positions of the relevant object or the relevant objects in the images;
taking into account the probability densities allocated in each case for the one or each relevant object to determine whether the one or more relevant objects were noticed in each case by the occupant, more especially the sum of the probability densities allocated in each case.

5. A method according to any of the preceding claims, further comprising, more especially if it is determined that one or more relevant objects in the surroundings of the vehicle were not noticed in each case by the occupant:
determining the central viewing direction of the occupant;
determining a desired central viewing direction of the occupant;
comparing the central viewing direction with the desired central viewing direction of the occupant;
if the comparison produces the fact that the central viewing direction and the desired central viewing direction do not coincide:
determining the change in the central viewing direction that the occupant has to carry out to change his central viewing direction to the desired central viewing direction;
outputting an indication of the change.

6. A method according to any of the preceding claims, further comprising:
more especially if it is determined that the one or more relevant objects in the surroundings of the vehicle were not noticed in each case by the occupant:
determining the course of the central viewing direction of the occupant;
determining the course of a desired central viewing direction of the occupant;
comparing the course of the central viewing direction with the course of the desired central viewing direction of the occupant;
determining an indication of a change of the central viewing direction that the occupant has to carry out to change his central viewing direction to the desired central viewing direction, taking into account the comparison of the course of the central viewing direction with the course of the desired central viewing direction of the occupant;
outputting the indication of the change.

7. A method according to any of the preceding claims, further comprising:
outputting an indication or a warning when it is determined that the relevant object or at least one of the relevant objects was not noticed.

8. A method according to any of the preceding claims, wherein the support device is spectacles or goggles.

9. A method according to claim 8, wherein the spectacles or goggles comprise a display, which is suitable to display contact-analogue views to the wearer of the spectacles or goggles.

10. A method according to claim 9, further comprising:
outputting an indication or a warning when it is determined that the relevant object, or at least one of the relevant objects, was not noticed;
wherein the indication or the warning more especially comprises one of the following:
emphasising the object(s), which were not noticed, in each case with the aid of an indication shown in a contact-analogue manner, more especially by a symbol or with a different transparency in comparison to the remaining view of the display with respect to the object(s), a different contrast or a specific colour.

11. A method according to claim 7, wherein the indication or the warning comprises:
a sound, more especially a sound seeming three-dimensional, with a volume, which is adapted to the warning or the indication, and a sound pattern; and/or
a vibration of the support device.

12. A device, comprising an electronic computing unit, wherein the device is set up to carry out a method according to any of claims 1 to 7.

13. A device according to claim 12, which is furthermore set up to cause the method steps according to any of claims 8 to 11 to be carried out.

14. A computer programme, comprising instructions for carrying out a method according to any of claims 1 to 11.

## Revendications

1. Procédé permettant de déterminer si un occupant d'un véhicule a remarqué un ou plusieurs objet(s) important(s) se trouvant dans l'environnement de ce véhicule comprenant des étapes consistant à :
recevoir des descriptions d'objets se trouvant dans l'environnement du véhicule qui ont été identifiés au moyen d'un système de capteurs de l'environnement du véhicule,
déterminer la présence d'un ou de plusieurs objet(s) important(s) se trouvant dans l'environnement du véhicule à partir des objets identifiés à l'aide du système de capteurs de l'environnement du véhicule,
recevoir une ou plusieurs prise(s) de vue d'une caméra qui est montée sur un dispositif porteur, en particulier une lunette qui, lors d'un port correct par un occupant du véhicule prend des vues qui comprennent des domaines de l'environnement du véhicule qui se trouvent dans le champ de vision instantané de l'occupant,
identifier des objets dans la ou les prise(s) de vue de la caméra,
déterminer si le ou les objet(s) important(s) situé(s) dans l'environnement du véhicule a(ont) été filmé(s) dans la ou les prise(s) de vue de la caméra,
déterminer si le ou les objet(s) important(s) situés dans l'environnement du véhicule ont été remarqués par l'occupant en se fondant sur le fait que le ou les objet(s) important(s) situé(s) dans l'environnement du véhicule a(ont) ou non été filmé(s) dans la ou les prise(s) de vue de la caméra, en supposant que le dispositif porteur est porté, de façon correcte par l'occupant du véhicule,
déterminer la position du ou des objet(s) important(s) dans la ou les prise(s) de vue de la caméra,
lors de la détermination du fait que le ou les objet(s) important(s) a(ont) ou non été remarqué(s) par l'occupant, la position du ou des objet(s) important(s) dans la ou les prise(s) de vue de la caméra étant prise en considération,
**caractérisé en ce que**
la détermination du fait que le ou les objet(s) important(s) a(ont) ou non été remarqué(s) par l'occupant comprend les étapes suivantes consistant à :
affecter des probabilités d'identification d'un objet par un occupant à des positions possibles d'un objet dans les prises de vue de la caméra, déterminer si le ou les objet(s) important(s) a(ont) ou non été respectivement identifié(s) par l'occupant en se fondant sur la probabilité affectée à la position de l'objet ou les probabilités respectivement affectées aux positions des objets.

2. Procédé conforme à la revendication 1,
selon lequel lors de la détermination du fait que le ou les objet(s) important(s) a(ont) ou non été remarqué(s) par un occupant on tient compte du fait que la position du ou des objet(s) important(s) est ou non respectivement située dans une zone située autour de la direction de visée centrale de l'occupant,
et on tient, en outre en particulier, compte du fait que la position du ou des objet(s) important(s) est ou non située pendant au moins une durée prédéfinie dans la zone située autour de la direction de visée centrale de l'occupant,
ce qui est en particulier déterminé à l'aide du nombre de prises de vue dans lesquelles la position du ou des objet(s) important(s) se trouve respectivement dans la zone située autour de la direction de visée centrale de l'occupant.

3. Procédé conforme à la revendication 1,
selon lequel les probabilités affectées décroissent entre le centre du champ de visée et les bords de ce champ de visée.

4. Procédé conforme à la revendication 1,
selon lequel plusieurs prises de vue, en particulier successives, de la caméra, sont reçues, et
la détermination du fait que le ou les objet(s) important(s) a(ont) ou non été respectivement remarqué(s) par l'occupant comprend les étapes suivantes consistant à :
affecter des densités de probabilité d'identification d'un objet par un occupant à des positions possibles d'un objet dans des prises de vue de la caméra,
déterminer pour le ou chacun des objet(s) important(s) les densités de probabilité qui sont respectivement affectées aux positions de l'objet important ou des objets importants dans les prises de vue,
prendre respectivement en considération les densités de probabilité affectées pour un ou chaque objet important de façon à déterminer si le ou les objet(s) important(s) a(ont) ou non été respectivement remarqué(s) par l'occupant, et en particulier la somme des densités de probabilité respectivement affectées.

5. Procédé conforme à l'une des revendications précédentes,
selon lequel, en particulier lorsqu'il a été déterminé que le ou les objet(s) important(s) situé(s) dans l'environnement du véhicule n'a(n'ont) respectivement pas été remarqué(s) par l'occupant,
on met en outre en oeuvre les étapes suivantes consistant à :
déterminer la direction de visée centrale de l'occupant,
déterminer la direction de visée centrale souhaitée de l'occupant,
comparer la direction de visée centrale à la direction de visée centrale souhaitée de l'occupant,
lorsqu'il résulte de la comparaison que la direction de visée centrale et la direction de visée souhaitée ne coïncident pas,
déterminer la modification de sa direction de visée centrale que doit ef-fectuer l'occupant pour pouvoir modifier sa direction de visée centrale de sorte qu'elle coïncide avec la direction de visée centrale souhaitée,
délivrer une indication concernant cette modification.

6. Procédé conforme à l'une des revendications précédentes, comprenant en outre des étapes consistant à :
en particulier dans le cas où il est déterminé qu'un ou plusieurs objet(s) important(s) se trouvant dans l'environnement du véhicule n'a(n'ont) respectivement pas été remarqué(s) par l'occupant,
déterminer les variations de la direction de visée centrale l'occupant,
déterminer les variations de la direction de visée centrale souhaitée de l'occupant,
comparer les variations de la direction de visée centrale et les variations de la direction de visée centrale souhaitée de l'occupant,
indiquer la modification de sa direction de visée centrale que l'occupant doit effectuer pour qu'elle coïncide avec la direction de visée centrale souhaitée, en prenant en considération la comparaison des variations de la direction de visée centrale avec les variations de la direction de vision centrale souhaitée de l'occupant,
délivrer une indication concernant cette modification.

7. Procédé conforme à l'une des revendications précédentes, comprenant en outre une étape consistant à :
délivrer une indication ou un avertissement lorsqu'il est déterminé que l'objet important ou au moins l'un des objets importants n'a pas été remarqué.

8. Procédé conforme à l'une des revendications précédentes,
selon lequel le dispositif porteur est des lunettes.

9. Procédé conforme à la revendication 8,
selon lequel les lunettes comportent un affichage susceptible d'afficher au porteur des lunettes des représentations analogiques de contact.

10. Procédé conforme à la revendication 9,
comprenant en outre des étapes consistant à :
délivrer une indication ou un avertissement lorsqu'il est déterminé que l'objet important ou au moins l'un des objets importants n'a pas été remarqué,
cette indication ou cet avertissement comprenant en particulier le fait de souligner le ou les objet(s) qui n'a(n'ont) respectivement pas été remarqué(s) à l'aide d'une indication représentée par analogie de contact, en particulier par un symbole ou une transparence comparativement différente de la partie restante de la représentation de l'affichage du ou des objet(s), un contraste différent ou une couleur déterminée.

11. Procédé conforme à la revendication 7,
selon lequel l'indication ou l'avertissement comprend :
une sonorité, en particulier une sonorité apparaissant de façon tridimensionnelle ayant une intensité sonore adaptée à l'avertissement ou à l'indication et un modèle de sonorité, et/ou
une vibration du dispositif porteur.

12. Dispositif comprenant une unité de calcul électronique permettant la mise en oeuvre d'un procédé conforme à l'une des revendications 1 à 7.

13. Dispositif conforme à la revendication 12,
permettant la mise en oeuvre des étapes du procédé conforme à l'une des revendications 1 à 11.

14. Programme d'ordinateur comprenant des instructions de code de programme permettant la mise en oeuvre du procédé conforme à l'une des revendications 1 à 11.
